(19) Europäisches Patentamt
European Patent Office
Office européen des brevets

(11) **EP 1 534 656 B1**

(12) **FASCICULE DE BREVET EUROPEEN**

(45) Date de publication et mention
de la délivrance du brevet:
**02.04.2008 Bulletin 2008/14**

(21) Numéro de dépôt: **03750519.5**

(22) Date de dépôt: **22.08.2003**

(51) Int Cl.:
*C07C 17/087* (2006.01)    *C07C 19/08* (2006.01)

(86) Numéro de dépôt international:
**PCT/EP2003/010083**

(87) Numéro de publication internationale:
**WO 2004/018394 (04.03.2004 Gazette 2004/10)**

(54) **PROCEDE DE FABRICATION DE PENTAFLUOROETHANE**

VERFAHREN ZUR HERSTELLUNG VON PENTAFLUORETHAN

PENTAFLUOROETHANE PRODUCTION METHOD

(84) Etats contractants désignés:
**AT BE BG CH CY CZ DE DK EE ES FI FR GB GR
HU IE IT LI LU MC NL PT RO SE SI SK TR**

(30) Priorité: **23.08.2002 FR 0210595**

(43) Date de publication de la demande:
**01.06.2005 Bulletin 2005/22**

(73) Titulaire: **SOLVAY (Société Anonyme)
1050 Bruxelles (BE)**

(72) Inventeurs:
• **PIEPHO, Eberhard
30627 Hannover (DE)**

• **WILMET, Vincent
B-1300 Wavre (BE)**
• **BUYLE, Olivier
B-1367 Autre-Eglise (BE)**

(74) Mandataire: **Jacques, Philippe et al
Solvay S.A.
Département de la Propriété Industrielle,
Rue de Ransbeek, 310
1120 Bruxelles (BE)**

(56) Documents cités:
**EP-A- 0 634 383          DE-C- 4 445 529**

**Description**

Le pentafluoroéthane (HFC-125) est un hydrofluoroalcane qui est utilisé, entre autres, dans des compositions réfrigérantes.

Il est connu de fabriquer du HFC-125 par hydrofluoration de précurseurs chlorés tels que le perchloroéthylène ou des hydrochlorofluoroéthanes. Au cours d'un tel procédé de fabrication, il n'est pas possible d'éviter la formation de chlorofluorocarbones (CFC), en particulier de CFC-115. Les CFC sont soupçonnés d'être impliqués dans la dégradation de la couche d'ozone stratosphérique. En conséquence, leur teneur dans le HFC-125 doit être réduite à l'issue du procédé de fabrication, ce qui peut s'avérer compliqué et coûteux.

La demande de brevet EP-A-634383 décrit entre autres la fabrication de HFC-125 à partir de tétrafluoroéthylène en présence d'un hydrofluorure de base azotée organique. La productivité en HFC-125 est de 40 mmol par heure et par litre de milieu réactionnel.

Il était souhaitable de mettre à disposition un procédé de fabrication de HFC-125 exempt de CFC-115, avec une productivité améliorée.

L'invention concerne dès lors un procédé pour la fabrication de pentafluoroéthane selon lequel on soumet du tétrafluoroéthylène à une réaction avec un hydrofluorure de base azotée organique à une température supérieure à 100° et ne dépassant pas 160°C.

Dans lé procédé selon l'invention, la température est souvent supérieure ou égale à 110°C. De préférence, la température est supérieure ou égale à 120°C. Une température supérieure ou égale à environ 130°C donne également de bons résultats. Dans le procédé selon l'invention, la température est souvent inférieure ou égale à 150°C. De préférence, la température est inférieure ou égale à 145°C. Une température d'environ 140°C donne également de particulièrement bons résultats.

Il a été trouvé de manière surprenante, que le procédé selon l'invention permet de fabriquer du HFC-125 exempt de CFC-115 au départ de tétrafluoroéthylène, dans des conditions de productivité particulièrement efficaces, sans formation de sous-produits ni dégradation de l'hydrofluorure de base azotée organique. En particulier, le procédé selon l'invention permet d'augmenter la productivité en HFC-125 par rapport à l'enseignement de l'état de la technique.

La productivité en HFC-125 du procédé selon l'invention est généralement supérieure ou égale à 0,05 mol/h par litre d'hydrofluorure de base azotée organique. Souvent elle est supérieure à 0,1 mol/h/l. Plus souvent elle est supérieure ou égale à 0,5 mol/h/l. La productivité en HFC-125 peut même être supérieure ou égale à 1 mol/h/l.

Dans le procédé selon l'invention, on met de préférence en oeuvre un hydrofluorure d'une base azotée organique répondant à la formule générale (I),

$$[B \cdot n\,HF] \qquad\qquad (I)$$

dans laquelle B représente une base azotée organique et n représente un nombre entier ou décimal $\leq 4$.

A titre de bases azotées B répondant à la formule (I), on peut mettre en oeuvre, par exemple, des amines y compris des hétérocycles azotés. Des exemples particuliers de bases azotées B répondent à la formule (II)

$$R1R2R3N \qquad\qquad (II)$$

dans laquelle les résidus R1, R2 et R3 peuvent être identiques ou différents et peuvent signifier
un atome d'hydrogène,
un résidu alkyle avec 1 à 20, de préférence 1 à 12, en particulier 1 à 6 atomes de carbone,
un résidu alcényle avec 2 à 20, de préférence 2 à 12, en particulier 2 à 6 atomes de carbone,
un résidu cycloalkyle avec 5 à 7 atomes de carbone,
un résidu cycloalcényle avec 5 à 7 atomes de carbone,
un résidu arylalkyle avec 7 à 10 atomes de carbone ou
un résidu aryle avec 6 à 10 atomes de carbone, qui peut encore être substitué par des groupes alkyles en C1-C3 ou alcoxy en C1-C3.
Sont préférés dans ce cas les résidus d'alkyle, de cycloalkyle, d'aryl-alkyle et d'aryle cités.

En outre, deux des résidus R1 à R3 peuvent former, ensemble avec l'atome d'azote qui les porte, un cycle à 5 à 7 membres, qui peut contenir un atome d'oxygène ou un atome d'azote supplémentaire. Ce cycle présente par conséquent 5 à 7 membres, dont un est l'atome d'azote et les autres sont, de préférence, des groupes CH$_2$. Un des groupes CH$_2$ peut également être remplacé par un atome d'oxygène ou d'azote, ce cas n'étant cependant pas préféré.

Les résidus R1 à R3 peuvent également former ensemble avec l'atome d'azote qui les porte, deux ou trois cycles à 5 à 7 membres, de préférence saturés, qui peuvent contenir des atomes d'azote supplémentaires, tels que par exemple dans l'hexaméthylènetétramine ou le diazabicyclooctane.

En outre, la base azotée B peut être un noyau hétérocyclique à 6 membres qui peut contenir un ou deux atomes d'azote et qui peut également être benzo-condensé, par exemple la pyridine, la pyrimidine ou la quinoline.

Des bases azotées B organiques particulièrement préférées sont les amines tertiaires, y compris les N-hétérocycles, avec en tout de 3 à 12 atomes de carbone. De préférence, l'amine tertiaire est choisie parmi la triméthylamine, la triéthylamine, la tri-n-propylamine, l'isopropyldiéthylamine, la tri-n-butylamirie, la N,N-diméthylaniline, la N-méthylpipéridine, la pyridine, la quinoline, la N,N'-tétraméthyléthylène-diamine et l'hexaméthylènetétramine. La tri n-butylamine et la triéthylamine sont particulièrement préférées. La triéthylamine est encore plus particulièrement préférée.

Le nombre n dans la formule générale (I) signifie la quantité molaire de HF par atome d'azote de la base B et représente un nombre entier ou décimal inférieur ou égal à 4. De préférence, n est un nombre inférieur ou égal à 3,5. De manière plus préférée, n est un nombre inférieur ou égal à 3. Le nombre n représente généralement un nombre supérieur ou égal à 0,5. De préférence, n est un nombre entier ou décimal, supérieur ou égal à 1, plus particulièrement supérieur ou égal à 1,5. De manière plus préférée, n est un nombre supérieur ou égal à environ 1,8. Un nombre n supérieur ou égal à 2 est plus particulièrement préféré.

Ci-dessous, on indique des exemples particuliers d'hydrofluorures de base azotée organique, répondant à la formule (I) pouvant être utilisés dans le procédé conforme à la présente invention :

$$[(CH_3)_3N \cdot 2,5\ HF]$$

$$[(C_2H_5)_3N \cdot 2,0\ HF]$$

$$[(C_2H_5)_3N \cdot 2,5\ HF]$$

$$[(C_2H_5)_3N \cdot 3,0\ HF]$$

$$[(n\text{-}C_3H_7)_3N \cdot 3,0\ HF]$$

$$[(i\text{-}C_3H_7)_2(C_2H_5)N \cdot 2,6\ HF]$$

$$[(n\text{-}C_4H_9)_3N \cdot 2,0\ HF]$$

$$[(n\text{-}C_4H_9)_3N \cdot 2,5\ HF]$$

$$[(n\text{-}C_4H_9)_3N \cdot 3,0\ HF]$$

$$[(CH_3)_2N\text{-}CH_2\text{-}CH_2\text{-}N(CH_3)_2 \cdot 4,7\ HF]$$

$$[(CH_2)_6N_4 \cdot 2\ HF]$$

Dans le procédé selon l'invention, l'utilisation de [triéthylamine · 2,5 HF], [triéthylamine · 2,0 HF] ou dé [tributylamine · 2,0 HF] est particulièrement préférée. La [triéthylamine · 2,5 HF] et la [triéthylamine · 2,0 HF] sont tout particulièrement préférées.

Les hydrofluorures répondant à la formule (I) utilisables dans le procédé selon l'invention peuvent être préparés par réaction directe des amines avec du HF.

Dans le procédé selon l'invention, la réaction entre le tétrafluoroéthylène et l'hydrofluorure de base azotée organique est généralement effectuée à une pression supérieure ou égale à 2 bar. De préférence, la pression est supérieure ou égale à 5 bar. Une pression supérieure ou égale à 7 bar est plus particulièrement préféré.

Dans le procédé selon l'invention, la réaction entre le tétrafluoroéthylène et l'hydrofluorure de base azotée organique est généralement effectuée à une pression inférieure ou égale à 30 bar. De préférence, la pression est inférieure ou égale à 25 bar. Une pression inférieure ou égale à 20 bar est plus particulièrement préférée.

Le procédé selon l'invention peut être mis en oeuvre, par exemple, dans une colonne à bulles qui peut être constituée d'un métal résistant à la corrosion, de verre de borosilicate ou de matière synthétique ou dans un autoclave réalisé en un matériau approprié. On préfère tout équipement résistant aux réactifs et aux pressions préférées et aux températures préférées décrits plus haut.

En général, l'addition d'un solvant n'est pas nécessaire. En cas de besoin, on peut toutefois aussi travailler en présence de quantités suffisantes d'un solvant aprotique, tel que le dioxane, le tétrahydrofuranne, l'acétonitrile ou la N-méthylpyrrolidone et la N-N-diméthylformamide.

Dans ce cas, la quantité de solvant n'excède généralement pas 80% en poids du poids total du milieu réactionnel. Souvent, la quantité de solvant n'excède pas 60% en poids du poids total du milieu réactionnel. Lorsqu'un solvant est mis en oeuvre, sa quantité est généralement d'au moins 20% en poids du poids total du milieu réactionnel.

Le procédé selon l'invention peut être réalisé en discontinu ou en continu. De bons résultats ont été obtenus en continu.

Lorsque le procédé selon l'invention est réalisé en discontinu, la durée de la réaction est généralement d'au moins 10 minutes. Souvent, la durée de la réaction est d'au moins 20 minutes. De préférence, elle est d'au moins 30 min. Dans ce cas, la durée de la réaction est généralement d'au plus 100 heures. Souvent, la durée de la réaction est d'au plus 30 heures. De préférence, elle est d'au plus 5 heures.

Lorsque le procédé selon l'invention est réalisé en continu, le temps de séjour, défini comme le rapport entre le volume du milieu réactionnel et le débit gazeux total à l'entrée du réacteur, est généralement supérieur ou égal à 5 min. Souvent, le temps de séjour est supérieur ou égal à 10 min. De préférence, le temps dé séjour est supérieur ou égal à 20 min. Le temps de séjour est généralement inférieur ou égal à 10 h. Souvent, le temps de séjour est inférieur ou égal à 5 h. De préférence le temps de séjour est inférieur ou égal à 3 h.

Au cours de la réaction, particulièrement lorsqu'elle est réalisée en continu, on peut contrôler le rapport entre la base azotée organique et le fluorure d'hydrogène dans l'hydrofluorure de base azotée organique. Ce contrôle peut être réalisé, par exemple, par un traitement d'au moins une fraction du milieu réactionnel avec du fluorure d'hydrogène, en particulier par des ajouts discontinus ou par un ajout en continu de fluorure d'hydrogène au milieu réactionnel.

Dans une variante, on peut soutirer une fraction du milieu réactionnel comprenant de l'hydrofluorure de base azotée organique, soumettre hors du réacteur au moins une partie de cette fraction à un traitement avec du fluorure d'hydrogène, puis recycler au moins une partie de la fraction traitée au milieu réactionnel.

Lorsqu'on contrôle le rapport entre la base azotée organique et le fluorure d'hydrogène dans l'hydrofluorure de base azotée organique, on vise généralement à ce que globalement, le nombre n dans l'hydrofluorure de base azotée organique dans le milieu réactionnel en cours de réaction n'atteigne pas une valeur inférieure à 70% de sa valeur initiale. Souvent, on effectue ce traitement de manière à ce que le nombre n n'atteigne pas une valeur inférieure à 80% de sa valeur initiale. De préférence, on veille à ce que le nombre n n'atteigne pas une valeur inférieure à 90% de sa valeur initiale.

Le cas échéant, on veille généralement à ce que le nombre n dans l'hydrofluorure de base azotée organique dans le milieu réactionnel en cours de réaction ne dépasse pas 150% de sa valeur initiale.

L'isolement du HFC-125 peut être effectué par exemple par distillation, ou par détente et par condensation.

Le HFC-125 obtenu selon le procédé selon l'invention est généralement directement totalement exempt de CFC-115.

L'invention concerne dès lors aussi un procédé de fabrication d'un mélange réfrigérant exempt de CFC-115 (chloropentafluoroéthane) selon lequel

(a) on fabrique du HFC-125 exempt de CFC-115 selon le procédé selon l'invention ;
(b) on mélange le HFC-125 avec au moins un autre hydrofluoroalcane choisi parmi le HFC-32 (difluorométhane), leHFC-134a (1,1,1,2-tétrafluoroéthane) et le HFC-143a (1,1,1-trifluoroéthane).

Dans un autre aspect, l'invention concerne une composition réfrigérante exempte de CFC-115 comprenant du HFC-125 et au moins un autre hydrofluoroalcane choisi parmi le HFC-32, le HFC-134a et le HFC-143a.

Dans encore un autre aspect l'invention concerne un procédé de fabrication d'un mélange de gaz stérilisant exempt de CFC-115 selon lequel

(a) on fabrique du HFC-125 exempt de CFC-115 selon le procédé selon l'invention
(b) on mélange le HFC-125 avec au moins du HFC-227ea (1,1,1,2,3,3,3-heptaflùoropropane et au moins un oxyde d'alkylène.

L'invention concerne aussi un mélange de gaz stérilisant exempt de CFC-115 comprenant du HFC-125, au moins du HFC-227ea et au moins un oxyde d'alkylène.

L'oxyde d'éthylène est préféré à titre d'oxyde alkylène.

Au sens de la présente invention on entend désigner par « exempt de CFC-15 » une teneur en CFC-115 inférieure ou égale à 10 mg/kg, voire 5 mg/kg. Par « totalement exempt de CFC-115 » on entend désigner une teneur en CFC-115 inférieure à la limite de détection CPV.

Les exemples suivants entendent illustrer le procédé selon l'invention sans toutefois le limiter.

Exemples 1 à 4

Dans un autoclave de 500 ml en acier Inox 316 équipé d'un agitateur, on a introduit 200g d'hydrofluorure de base azotée indiqué dans le tableau ci-dessous et environ 2 g de limonène. Le système a été chauffé à la température de réaction et la quantité indiquée de tétrafluoroéthylène pur a été introduite de manière à atteindre la pression initiale de la réaction. On a effectué des prélèvements en phase liquide et gaz afin de déterminer le rendement en HFC-125 par analyse CPV (chromatographie en phase vapeur).

TFEe : tétrafluoroéthylène mis en oeuvre

| Exemple | Hydrofluorure de base azotée organique | T (°C) | TFEe (g) | Pression initiale (bar) | Productivité en HFC-125 (mol par heure et par litre d'hydrofluorure de base azotée organique) |
|---------|----------------------------------------|--------|----------|-------------------------|----------------------------------------------------------------------------------------------|
| 1 | [$(C_2H_5)_3N \cdot 2,0$ HF] | 120 | 9 | 10,9 | 0,76 |
| 2 | [$(C_2H_5)_3N \cdot 2,0$ HF] | 140 | 8,5 | 12,8 | 1,70 |
| 3 | [$(C_2H_5)_3N \cdot 2,5$ HF] | 120 | 9,4 | 10 | 0,47 |
| 4 | [$(n-C_4H_9)_3N \cdot 2,0$ HF] | 120 | 8,7 | 10 | 0,18 |

Le HFC-125 obtenu ne contenait pas de CFC-115. Aucune décomposition de l'hydrofluorure de base azotée n'a été observée.

Exemple 5

Dans un autoclave de 500 ml, on a introduit 200 ml de complexe $(n-(C_4H_9)_3N.2,5HF$. Le système a été chauffé à 120°C et du tétrafluoroéthylène (37% en volume, dilué dans de l'hélium) a été introduit en continu. Le débit d'alimentation était de 92 mmol de tétrafluoroéthylène/heure. La pression était de 20 bar. Le temps de séjour était de 2h. La phase gaz sortant du réacteur a été analysée par CPV (chromatographie en phase vapeur). La productivité en HFC-125 était de 0.17 mol par heure et par litre de complexe.
L'essai a été poursuivi pendant 550 h sans signe de désactivation en ajoutant régulièrement du HF pour maintenir un rapport molaire HF/$(n-C_4H_9)_3N$ moyen de 2,4.
Aucun sous-produit n'a été détecté au cours de l'essai. Le produit obtenu était exempt de CFC-115.

**Revendications**

1. Procédé pour la fabrication de pentafluoroéthane selon lequel on soumet du tétrafluoroéthylène à une réaction avec un hydrofluorure de base azotée organique à une température supérieure à 100°C et ne dépassant pas 160°C.

2. Procédé selon la revendication 1 dans lequel la température est de 110 à 150°C.

3. Procédé selon la revendication 2 dans lequel la température est de 120 à 140°C.

4. Procédé selon l'une quelconque des revendications 1 à 3, dans lequel la pression est maintenue de 2 à 30 bar.

5. Procédé selon l'une quelconque des revendications 1 à 4, dans lequel l'hydrofluorure de base azotée organique répond à la formule générale [B*nHF] dans laquelle B désigne la base azotée organique et n désigne un nombre entier ou décimal inférieur ou égal à 4.

6. Procédé selon la revendication 5 dans lequel n désigne un nombre inférieur ou égal à 3.

7. Procédé selon la revendication 5 ou 6 dans lequel n désigne un nombre supérieur ou égal à 2.

8. Procédé selon l'une quelconque des revendications 1 à 7, dans lequel la base azotée organique est choisie parmi la triéthylamine et la tri n-butylamine.

9. Procédé selon la revendication 8, dans lequel la base azotée organique est la triéthylamine.

10. Procédé selon l'une quelconque des revendications 1 à 9, dans lequel la réaction est réalisée en continu.

11. Procédé de fabrication d'un mélange réfrigérant exempt de CFC-115 (chloropentafluoroéthane) selon lequel

   (a) on fabrique du pentafluoroéthane (HFC-125) exempt de CFC-115, selon le procédé selon l'une quelconque des revendications 1 à 10;
   (b) on mélange le HFC-125 obtenu avec au moins un autre hydrofluoroalcane choisi parmi le HFC-32 (difluorométhane), le HFC-134a (1,1,1,2-tétrafluoroéthane) et le HFC-143a (1,1,1-trifluoroéthane).


**Claims**

1. Process for the manufacture of pentafluoroethane, according to which tetrafluoroethylene is subjected to reaction with an organic nitrogenous base hydrofluoride at a temperature of greater than 100°C and not exceeding 160°C.

2. Process according to Claim 1, in which the temperature is from 110 to 150°C.

3. Process according to Claim 2, in which the temperature is from 120 to 140°C.

4. Process according to any one of Claims 1 to 3, in which the pressure is maintained from 2 to 30 bar.

5. Process according to any one of Claims 1 to 4, in which the organic nitrogenous base hydrofluoride corresponds to the general formula [B*nHF] in which B denotes the organic nitrogenous base and n denotes a whole or decimal number of less than or equal to 4.

6. Process according to Claim 5, in which n denotes a number of less than or equal to 3.

7. Process according to Claim 5 or 6, in which n denotes a number of greater than or equal to 2.

8. Process according to any one of Claims 1 to 7, in which the organic nitrogenous base is chosen from triethylamine and tri(n-butyl)amine.

9. Process according to Claim 8, in which the organic nitrogenous base is triethylamine.

10. Process according to any one of Claims 1 to 9, in which the reaction is carried out continuously.

11. Process for the manufacture of a refrigerant mixture devoid of CFC-115 (chloropentafluoroethane), according to which

   (a) pentafluoroethane (HFC-125) devoid of CFC-115 is manufactured according to the process according to anyone of claims 1 to 10;
   (b) the HFC-125 obtained is mixed with at least one other hydrofluoroalkane chosen from HFC-32 (difluoromethane), HFC-134a (1,1,1,2-tetrafluoroethane) and HFC-143a (1,1,1-trifluoroethane).


**Patentansprüche**

1. Verfahren zur Herstellung von Pentafluorethan, gemäß dem man Tetrafluorethylen einer Umsetzung mit einem Hydrofluorid einer organischen Stickstoffbase bei einer Temperatur von mehr als 100°C und höchstens 160°C unterwirft.

2. Verfahren nach Anspruch 1, bei dem die Temperatur 110 bis 150°C beträgt.

3. Verfahren nach Anspruch 2, bei dem die Temperatur 120 bis 140°C beträgt.

4. Verfahren nach einem der Ansprüche 1 bis 3, bei dem man den Druck bei 2 bis 30 bar hält.

5. Verfahren nach einem der Ansprüche 1 bis 4, bei dem das Hydrofluorid der organischen Stickstoffbase der allgemeinen Formel [B*nHF], worin B für die organische Stickstoffbase steht und n für eine ganze oder gebrochene Zahl kleiner gleich 4 steht, entspricht.

**6.** Verfahren nach Anspruch 5, bei dem n für eine Zahl kleiner gleich 3 steht.

**7.** Verfahren nach Anspruch 5 oder 6, bei dem n für eine Zahl kleiner gleich 2 steht.

**8.** Verfahren nach einem der Ansprüche 1 bis 7, bei dem man die organische Stickstoffbase unter Triethylamin und Tri-n-butylamin auswählt.

**9.** Verfahren nach Anspruch 8, bei dem es sich bei der organischen Stickstoffbase um Triethylamin handelt.

**10.** Verfahren nach einem der Ansprüche 1 bis 9, bei dem man die Umsetzung kontinuierlich durchführt.

**11.** Verfahren zur Herstellung einer CFKW-115 (Chlorpentafluorethan) freien Kältemittelmischung bei dem man

(a) CFKW-115- freien Pentafluorethan (HFKW-125) nach dem Verfahren gemäß einem der Ansprüche 1 bis 10 herstellt
(b) das erhaltene HFKW-125 mit mindestens einem anderen Hydrofluoroalkan, ausgewählt aus HFKW-32 (Difluormethan), HKFW-134a (1,1,1,2-Tetrafluorethan) und HFKW-143a (1,1,1-Trifluorethan) mischt.

**EP 1 534 656 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 634383 A